# EUROPÄISCHE PATENTSCHRIFT

(11) **EP 3 603 745 B1**
(45) Veröffentlichungstag und Bekanntmachung des Hinweises auf die Patenterteilung: **22.12.2021**
(21) Anmeldenummer: 18186381.2
(22) Anmeldetag: 30.07.2018
(51) Int. Cl.: A61N 5/06

(54) **THERAPIEGERÄT ZUR BESTRAHLUNG EINER OBERFLÄCHE EINES GEWEBES**
THERAPY DEVICE FOR IRRADIATING A SURFACE OF A TISSUE
APPAREIL THÉRAPEUTIQUE DESTINÉ À IRRADIER UNE SURFACE D'UN TISSU

(43) Veröffentlichungstag der Anmeldung: 05.02.2020
(73) Patentinhaber: bredent medical GmbH & Co. KG, 89250 Senden (DE)
(72) Erfinder: MICKO, Gerald, 89079 Wiblingen (DE); VANDER BEKEN, Seppe, 89134 Blaustein (DE); STOCK, Karl, 73479 Ellwangen (DE); MADER, Raphael, 89079 Ulm (DE)
(74) Vertreter: Baur & Weber Patentanwälte PartG mbB

(56) Entgegenhaltungen:
- EP-A1- 3 202 359
- US-A1- 2004 111 132
- US-A1- 2015 112 411
- US-A1- 2017 182 191

## Beschreibung

Die Erfindung betrifft ein Therapiegerät zur Bestrahlung einer Oberfläche eines Gewebes, insbesondere Hautgewebe.

Aus dem allgemeinen Stand der Technik ist die Verwendung von Lichtquellen zu therapeutischen Zwecken bekannt. Ein Beispiel dafür ist die Verwendung einer Lichtquelle zur antimikrobiellen photodynamischen Therapie, die in der zahnärztlichen Praxis zur Behandlung von bakteriellen Infektionen auf vielfältige Weise eingesetzt wird. Die antimikrobielle photodynamische Therapie wird u.a. zur Reduktion von paradontalpathogenen Bakterien eingesetzt, so dass in der Parodontitistherapie eine ergänzende Behandlungsmöglichkeit geschaffen wurde. Dazu bedarf es einer Lichtquelle, die in einem passenden Wellenlängenbereich oder mit einer passenden Wellenlänge eine lichtinduzierte Inaktivierung von Zellen, Mikroorganismen, Molekülen oder dergleichen durchführen kann. Die antimikrobielle photodynamische Therapie erfolgt durch die Umsetzung von Umgebungssauerstoff in reaktiven Sauerstoffspezies mittels eines Photosensibilisators, welcher die photochemische Energieübertragung bewirkt.

Außerhalb des Dentalbereichs ist aber auch die Verwendung für die Hautbehandlung bei Vorliegen von Wundverletzungen an sich bekannt.

So ist aus der EP 2 440 287 B1 eine Vorrichtung für die photodynamische Therapie und/oder zur Abtötung oder Reduktion von Mikroorganismen, bekannt, enthaltend eine Bestrahlungseinheit mit wenigstens einer Lichtquelle, mittels welcher ein auf ein zu therapierendes Wundareal aufgebrachter Photosensibilisator durch Bestrahlung aktiviert wird, ferner enthaltend eine in der Bestrahlungseinheit angeordnete Kamera zur Erfassung von Bildern der Wunde sowie eine Positioniereinrichtung, mittels welcher die Bestrahlungseinheit zum Wundareal ausrichtbar ist. Es wird vorgeschlagen, dass die Lichtquelle in der Bestrahlungseinheit mittels einer Führungseinrichtung und mittels einer Antriebseinheit bewegbar und sequentiell auf wenigstens zwei unterschiedliche Bestrahlungspositionen des Wundareals positionierbar sind, dass dem mittels der Kamera erfassten und auf einem Display dargestellten Kamerabild ein eingeblendetes Raster überlagert ist, welches den Bestrahlungspositionen der Lichtquelle entspricht, und dass das Display derart ausgebildet ist, dass die mittels der Lichtquellen zu bestrahlenden Bestrahlungsfelder mit Markierungsmitteln im Display markierbar sind.

Neben der antimikrobiellen photodynamischen Therapie sind aber auch noch andere therapeutische Konzepte bekannt, die unter Einwirkung einer Lichtquelle durchgeführt werden. Als Beispiel sei hier die regenerative Photobiomodulationstherapie genannt, die beispielsweise im Nah-InfrarotBereich durchgeführt werden kann und eine verbesserte Abheilung von Wundgewebe durch entzündungshemmende Wirkung schafft.

Zu anderen therapeutischen oder kosmetischen Zwecken wurden ebenfalls Bestrahlungsgeräte mit Lichtquellen eingesetzt, die auf eine Hautoberfläche gerichtet sind.

Aus der DE10 2015 226 377 A1 ist beispielsweise eine Bestrahlungseinheit zum Bereitstellen von Strahlungspulsen zum Bestrahlen einer Hautoberfläche, insbesondere zur Epilation, bekannt, wobei die Bestrahlungseinheit eine Lichtquelleneinheit aufweist, die dazu ausgelegt ist, die Strahlungspulse mit vorgebbarer Pulsdauer, mit vorgebbarer Strahlungsleistung und in einem vorgebbaren zeitlichen Pulsabstand bereitzustellen, wobei die Lichtquelleneinheit weiterhin dazu ausgelegt ist, einen Bereich mit vorbestimmter Größe in vorbestimmtem Abstand zur Lichtquelleneinheit in einer Hauptabstrahlrichtung der Lichtquelleneinheit zu beleuchten. Die Lichtquelleneinheit weist mindestens eine Festkörperlichtquelle auf und die Bestrahlungseinheit weist eine Sensoreinheit eine Steuereinrichtung auf, um die mindestens eine Festkörperlichtquelle anzusteuern.

Aus der WO 2004/064923 A1 ist eine Vorrichtung zur kosmetischen Verbesserung eines von Akne betroffenen Hautbereichs bekannt, bei der Lichtstrahlung von einer Beleuchtungseinrichtung auf die Haut gerichtet wird. Das Gerät weist eine Steuereinheit auf, die eine oder mehrere LEDs der Beleuchtungseinrichtung betätigt. Während einer Zeit von mindestens 100ms erhält die Haut Lichtenergie von den LEDs, die eine photochemische Reaktion hervorruft, die die Produktion von freien Radikalen (Singulett-Sauerstoff) stimuliert, die mit Bakterien reagieren und diese zumindest teilweise deaktivieren oder zerstören, die zu den Symptomen des Hautzustandes beitragen, wobei keine photothermische Reaktion in der Haut stattfindet. Die Dauer einer Einzeldosis kann bis zu 10 Stunden dauern, z.B. bei einer Behandlung über Nacht.

Aus der FR 2940915 A1 ist eine Vorrichtung zur Behandlung von menschlichen keratinischen Materialien, insbesondere der Haut oder des Haares, bekannt. Die Vorrichtung umfasst einen Träger, der ein photokatalytisches Material trägt und so konfiguriert ist, dass er mit den keratinischen Materialien in Kontakt kommt. Des weiteren ist eine erste Lichtquelle vorgesehen, die so konfiguriert ist, dass sie das photokatalytische Material beleuchtet, um in Kontakt mit dem Material vorhandene Verunreinigungen einer photokatalytischen Reaktion auszusetzen, und eine zweite Lichtquelle vorgesehen, die sich von der ersten unterscheidet und so konfiguriert ist, dass sie Licht im Wesentlichen in einem Bereich um eine dominante Wellenlänge emittiert und eine kosmetische oder therapeutische Wirkung auf die behandelten keratinischen Materialien hat.

Vorrichtungen, bei denen ein Laserstrahl in einer Scanbewegung über einen begrenzten Bereich auf einer Hautoberfläche geführt wird, sind beispielsweise aus der EP 1 418 984 B1 oder der WO 2009/088550 A2 bekannt. Therapiegeräte sind auch aus US 2015/112411 A1 und US 2004/0111132 A1 bekannt. Um möglichst vielfältige phototherapeutische Anwendungen bei der Wundbehandlung zu schaffen, ist eine konstante Ausleuchtung der zu behandelnden Oberfläche bei gleichzeitig therapeutisch wirksamer bzw. indizierter Leistungsdichte notwendig. Dabei sollen auch verschiedene Phototherapiearten kombinierbar sein.

Die EP 3202359 A1 zeigt eine Hautbehandlungsvorrichtung, insbesondere eine Vorrichtung zur temporalen Haarentfernung, mit einer Lichtemissionseinheit mit einem Substrat und ersten LEDs zum Emittieren von Licht mit einer ersten Spitzenemissionswellenlänge und zweiten LEDs zum Emittieren von Licht mit einer zweiten Spitzenemissionswellenlänge, die sich von der ersten Spitzenemissionswellenlänge unterscheidet. Die erste Wellenlänge kann zur Haarentfernung und die zweite zur Hautverjüngung ausgewählt werden. Jede LED kann individuell gesteuert werden. Die Abstrahlung wird nicht lateral begrenzt, sodass sich das Licht verschiedener LEDs mischt.

Die US 2017/0182191 A1 beschreibt ein Verfahren zur kontrollierbaren Behandlung von Schmerzen im afferenten Nervensystem eines Patienten mit einer Zielgewebestruktur, die genetisch so modifiziert wurde, dass sie ein lichtempfindliches Protein aufweist. Das dafür verwendete Lichtzuführungselement ist so konfiguriert, dass es Strahlung auf mindestens einen Teil einer Zielgewebestruktur richtet. Im Lichtzuführungselement können optische Elemente hinzugefügt werden, um die Strahlbreite und Divergenz zu verändern und die Bestrahlungsstärke des Lichts, das die Zielebene erreicht, zu verbessern.

Es ist daher Aufgabe der Erfindung, ein Therapiegerät zur Bestrahlung einer Oberfläche eines Gewebes, insbesondere Hautgewebe zu schaffen, die eine einfache Anpassung an verschiedene Therapiearten, ermöglicht.

Diese Aufgabe wird durch den unabhängigen Patentanspruch 1 gelöst. Weitere vorteilhafte Ausgestaltungen der Erfindung sind jeweils Gegenstand der Unteransprüche. Diese können in technologisch sinnvoller Weise miteinander kombiniert werden. Die Beschreibung, insbesondere im Zusammenhang mit der Zeichnung, charakterisiert und spezifiziert die Erfindung zusätzlich.

Gemäß der Erfindung wird ein Therapiegerät zur Bestrahlung einer Oberfläche eines Gewebes, insbesondere Hautgewebe angegeben, das in einer Mehrfachanordnung mehrere erste LEDs mit einer ersten zentralen Wellenlänge und mehrere zweite LEDs mit einer zweiten zentralen Wellenlänge aufweist, wobei die erste zentrale Wellenlänge zur Durchführung einer ersten Phototherapieart und die zweite zentrale Wellenlänge zur gleichzeitigen Durchführung einer zweiten Phototherapieart auf dem Gewebe geeignet sind, wobei die mehreren ersten LEDs und die mehreren zweiten LEDs separat bezüglich ihrer Leistung und Bestrahlungsdauer als elektrische Betriebsgrößen steuerbar sind und jede erste LED und jede zweite LED jeweils ein optisches Modul zur lateralen Begrenzung der Abstrahlung in einem Belichtungsfeld zugeordnet ist, so dass den mehreren ersten LEDs erste Belichtungsfelder und den mehreren zweiten LEDs zweite Belichtungsfelder zugeordnet sind, wobei sich die ersten Belichtungsfelder und die zweiten Belichtungsfelder auf der Oberfläche des Gewebes zu einem gemeinsamen Bestrahlungsgebiet überlagern, so dass jedes erste Belichtungsfeld mit einer Leistungs- und Energiedichte oberhalb einer Therapieschwelle indiziert für die erste Phototherapieart und jedes zweite Belichtungsfeld mit einer Leistungs- und Energiedichte oberhalb einer Therapieschwelle indiziert für die zweite Phototherapieart mit möglichst homogenen Leistungsverteilungen bestrahlbar ist.

Demnach wird ein Therapiegerät geschaffen, bei der die erste zentrale Wellenlänge eine erste Phototherapieart ausführen kann und die zweite zentrale Wellenlänge zur Durchführung einer zweiten Phototherapie als zweite Therapieform geeignet ist. Jeder ersten bzw. zweiten LED ist dabei ein Belichtungsfeld zugeordnet, so dass eine flächige Bestrahlung auf der Oberfläche des Hautgewebes mittels der beiden zentralen Wellenlängen jeweils für jedes der Belichtungsfelder individuell zuschaltbar ist. Die Vielzahl der ersten bzw. zweiten Belichtungsfelder ergänzen sich auf der Oberfläche des Gewebes zu einem gemeinsamen Bestrahlungsgebiet, so dass für jedes Belichtungsfeld ausgewählt werden kann, ob die dazugehörige LED aktiviert werden soll oder nicht. Das erfindungsgemäße Therapiegerät sieht vor, dass je nach Anwendungsfall eine unterschiedliche Anzahl von ersten und zweiten Belichtungsfeldern gewählt werden kann, so dass eine unterschiedliche Anzahl erster bzw. zweiter LEDs zur Bestrahlung des gemeinsamen Bestrahlungsgebiets vorgesehen werden müssen. Somit kann es Gebiete auf der Oberfläche des Hautgewebes geben, die nur von der ersten zentralen Wellenlänge, nur von der zweiten zentralen Wellenlänge, sowohl von der ersten als auch der zweiten zentralen Wellenlänge oder von keiner der zentralen Wellenlängen bestrahlt werden. Dies ermöglicht es, in unterschiedlichen Bereichen unterschiedliche Therapiearten anzuwenden. Somit besteht ein zentraler Gedanke der Erfindung darin, ein Behandlungsgebiet in eine Vielzahl von Belichtungsfeldern aufzuteilen, so dass das gemeinsame Bestrahlungsgebiet bezüglich Bestrahlung mit der ersten bzw. der zweiten zentralen Wellenlänge frei gewählt werden kann. Die Bestrahlungsdauern und Leistungsdichten werden dabei so gewählt, dass für jedes Belichtungsfeld im Falle einer Aktivierung der zugehörigen LED eine der Phototherapieart-Auswahl entsprechende Schwelle erreicht werden kann.

Gemäß Ausführungsformen der Erfindung ist die erste Phototherapieart eine regenerative Photobiomodulationstherapie oder eine photodynamische Therapie und die zweite Phototherapieart eine weitere regenerative Photobiomodulationstherapie.

So kann beispielsweise beim Einsatz in der Wundbehandlung mittels der ersten zentralen Wellenlänge ein Wundareal zur Desinfektion bestrahlt werden, das vorher auf der Oberfläche des zu bestrahlenden Gewebes mit einem entsprechenden photosensitiven Wirkstoff, beispielsweise Thiazinfarbstoffe, benetzt würde. Die Wellenlänge der ersten LEDs wird dabei im Aktivierungsbereich des photosensitiven Materials gewählt werden. Die Wellenlänge der zweiten LEDs soll dabei so gewählt sein, dass das Licht der zweiten LED den Photosensibilisator durchdringen kann. Somit kann in denjenigen Gebieten, bei denen sowohl die erste als auch die zweite zentrale Wellenlänge auf die Oberfläche des zu bestrahlenden Gewebes trifft, eine Photobiomodulationstherapie des Wundgrundes zusätzlich zur oberflächigen antimikrobiellen photodynamischen Therapie stattfinden. Für die zweite LED wird eine Wellenlänge im Nahinfrarotbereich gewählt werden, da häufig verwendete Photosensibilisatoren in diesem spektralen Bereich nicht absorbieren und dieser für eine Photobiomodulationstherapie geeignet ist.

Außerhalb derjenigen Wundflächen, die vermutlich von pathogenen Keimen kolonisiert sind und wofür die antimikrobielle photodynamische Therapie indiziert ist, kann zur besseren Wundheilung oder Geweberegeneration lediglich die zweite zentrale Wellenlänge über die zweiten LEDs aktiviert sein. Darüber hinaus ist es sogar denkbar, auch die erste zentrale Wellenlänge über die ersten LEDs, ohne Verwendung eines Photosensibilisators, an für die Photobiomodulationstherapie geeigneten Bestrahlungsparameter zu wählen oder unterstützend zur zweiten zentralen Wellenlänge zuschalten. Dabei wären die beiden Photobiomodulationstherapieformen bezüglich ihrer Eindringtiefe in das Gewebe unterschiedlich, so dass aufgrund der unterschiedlichen Wirkungen hier von zwei Phototherapiearten gesprochen wird. So können z.B. in der ersten Therapieform Entzündungen behandelt werden und in der zweiten Therapieform eine Schmerzbehandlung durchgeführt werden.

Therapeutische Indikationen sind unter anderem nicht-neoplastische dermatologische Erkrankungen wie akute und chronische Wunden, Geschwüre, Abszesse, Blasen und Verbrennungen, bakterielle, virale und dermatophytische Hauterkrankungen, lokalisierte Sklerodermie und Onychomykose oder auch dermato-onkologische Erkrankungen wie aktinische Keratose, Bowen-Krankheit, in situ Plattenepithelkarzinom und Basalzellkarzinom.

Gemäß einer Ausführungsform der Erfindung sind für jedes Belichtungsfeld mehrere erste und/oder mehrere zweite LEDs vorgesehen.

Um die zur photodynamischen Therapie notwendigen Energiedichten bei gleichzeitig möglichst homogener Abstrahlcharakteristik erreichen zu können, kann es notwendig sein, mehrere erste LEDs für jedes erste Belichtungsfeld vorzusehen. Typische Bestrahlungsleistungen bewegen sich hier im Bereich von 30 bis 80 mW/cm². Für eine Bestrahlungszeit von beispielsweise maximal 10 Minuten bei einer benötigten Energiedichte von 24 J/cm² ergibt sich eine Bestrahlungsleistung von 40 mW/cm², welche sicher angewandt werden kann, ohne dass dabei die Hauttemperatur signifikant ansteigt, auch nicht bei Bestrahlung auf hochkonzentrierte Methylenblau-gefärbte Haut. Eine zu hohe Leistungsdichte und damit verbundene Erhöhung der Hauttemperatur stellt vor allem bei Patienten mit schlechter Durchblutung ein Risiko dar, welches ggf. die Wundheilung beeinträchtigen könnte. Die Anzahl und Bestrahlungsparameter der zweite LEDs sollen dabei so gewählt werden, dass innerhalb der gleichen Bestrahlungszeit die für die Wirksamkeit notwendige Energiedosis eingehalten und nicht unter- oder überschritten werden kann. Typische Energiedosen bei der Photobiomodulationstherapie bewegen sich im Bereich 0,5 bis 25 J/cm².

Die ersten Belichtungsfelder und die zweiten Belichtungsfelder sind in Form einer Mehrfachanordnung ausgebildet.

Vorzugsweise werden die Belichtungsfelder als zweidimensionale Anordnung ausgebildet sein, so dass die Oberfläche des Gewebes in einer Art zweidimensionalen Matrix wahlweise mit den unterschiedlichen zentralen Wellenlängen bestrahlt werden kann. Wie bereits oben erwähnt, ist es nicht zwingend erforderlich, die Zahl der Zeilen bzw. Spalten für jede Mehrfachanordnung der ersten Belichtungsfelder genau gleich zu derjenigen Mehrfachanordnung der zweiten Belichtungsfelder zu wählen.

Gemäß der Erfindung sind die ersten LEDs und die zweiten LEDs auf einem Bauteileträger angeordnet, dessen Außenabmessung im Wesentlichen der zu bestrahlenden Oberfläche auf dem Gewebe entspricht.

Üblicherweise beträgt der Abstand zwischen dem Bauteileträger im Therapiegerät zu der zu bestrahlenden Oberfläche auf dem Gewebe etwa 3 bis 30 cm, ein typischer Wert beträgt ungefähr 15 cm. Das zweidimensional ausgebildete Behandlungsgebiet weist Seitenlängen auf, die in etwa in einem ähnlichen Bereich, d. h. zwischen 3 und 30 cm liegen können. Der Bauteileträger überdeckt dabei bezüglich seiner Außenabmessung das zu bestrahlende Gebiet. Jede LED ist zwar mit einem optischen Modul versehen, dieses dient jedoch der Abbildung der Emitterfläche einer einzelnen LED auf das der LED zugewiesene Belichtungsfeld. Das optische Modul ist dazu vorgesehen, die Emitterfläche einer einzelnen LED auf das der LED zugewiesene Belichtungsfeld, nicht jedoch für eine Projektion auf ein noch größeres Bestrahlungsgebiet, so dass die Abmessungen der Belichtungsfelder dem Raster der LEDs folgt.. Aufgrund dieser Vorgehensweise wird der Aufbau eines Therapiegeräts deutlich vereinfacht, wobei zusätzlich auch die Homogenität über das Behandlungsgebiet verbessert wird.

Dabei kann an ausgewählten Belichtungsfeldern, insbesondere im Bereich der zentralen Belichtungsfeldern bezüglich der zu bestrahlenden Oberfläche, die ersten LEDs und die zweiten LEDs so angeordnet sein, dass ein Freiraum im Bauteileträger gebildet wird, der vorzugsweise zum Anbringen einer Kamera genutzt werden kann. Dabei kann eine Anzeigeeinheit vorgesehen sein, die beispielsweise an einer Oberseite des Therapiegeräts angeordnet ist und bei Gebrauch ausgeklappt werden kann, so dass Daten der Kamera auf der Anzeigeeinheit dargestellt werden.

Gemäß dieser Vorgehensweise ist es möglich, einem Behandler entsprechende Hilfsmittel zur Verfügung zu stellen, so dass eine Überwachung, beispielsweise bezüglich des Abstands des Therapiegeräts von der zu bestrahlenden Oberfläche und der Auswahl der Belichtungsfelder bezüglich emittieren von Licht der ersten zentralen Wellenlänge bzw. zweiten zentralen Wellenlänge, möglich ist. Die Anzeigeeinheit kann dabei auf fachübliche Weise auch als Eingabeeinheit ausgebildet sein, wie dies üblicherweise in Form von berührungssensitiven Bedienoberflächen bekannt ist. Somit kann auf der Anzeigeeinheit nicht nur das Bild der Kamera dargestellt werden, sondern auch eine Auswahl der zu bestrahlenden Belichtungsfelder oder generell eine Auswahl von Parameterwerten oder Programmabläufen getroffen werden.

Um den Freiraum im Bauteileträger zu bilden, können die LEDs beispielsweise im Bereich der zentralen Belichtungsfelder an anderen Positionen angeordnet sein. Falls an diesen Belichtungsfeldern jedoch mehrere LEDs des gleichen Typs verwendet werden, ist es auch vorgesehen, diese in einem anderen Bestückungsmuster anzuordnen. So kann es vorgesehen sein, die LEDs nebeneinander liegend in Form eines Streifens anzuordnen, während im Bereich des Freiraums die LEDs L-förmig oder U-förmig angeordnet werden, so dass diese um den Freiraum herum anbringbar sind. Das optische Modul soll dabei für LEDs in diesen Bereichen entsprechend angepasst werden, damit die abweichende LED-Positionierung auf dem Bauteileträger die Regelmäßigkeit bei der Mehrfachanordnung der Bestrahlungsfelder und die Homogenität der Lichtleistung auf Therapieebene nicht zerstört.

Gemäß einer weiteren Ausführungsform der Erfindung weist das optische Modul der ersten und/oder zweiten LEDs eine asphärische Linse auf, die vorzugsweise als Kunststofflinse gefertigt ist.

Mittels dieser Ausgestaltungen der optischen Module ist eine homogene Ausleuchtung der jeweiligen Belichtungsfelder möglich, wobei als Kunststofflinse gefertigte Linsen der optischen Module für die ersten und/oder zweiten LEDs auch in einer Linsenmatrix zusammengefasst sein können.

Zusätzlich kann eine zuschaltbare Lichtquelle zur optischen Kontrolle durch den Behandler der zu behandelnden Oberfläche vorgesehen sein. Die zuschaltbare Lichtquelle wird besonders vorteilhaft weißes Licht abstrahlen, so dass eine manuelle Ausrichtung des Therapiegeräts relativ zum zu behandelnden Gebiet leichter durchführbar ist. Die als Weißlicht-LED ausgeführte Lichtquelle kann am Rand des Gehäuses des Therapiegeräts angebracht werden, um störende Reflektionen von einem die Unterseite überdeckenden transparenten Fenster zu vermeiden.

Gemäß einer weiteren Ausführungsform der Erfindung weist das Therapiegerät einen Abstandssensor zur Messung eines Abstands zur Oberfläche des zu behandelnden Gewebes auf.

Gemäß der Erfindung sind die ersten LEDs und/oder die zweiten LEDs bezüglich ihrer Leistungsabgabe steuerbar. Dabei können die Leistungsabgabe und der Einschaltzustand der ersten LEDs und/oder der zweiten LEDs in einem Programmspeicher als Therapiedaten hinterlegt sein.

Demnach ist es möglich, die Leistungsabgabe sowie über den Einschaltzustand das zugehörige Belichtungsfeld sowohl für die ersten LEDs als auch die zweiten LEDs individuell zu steuern. Dabei kann typischerweise patientenindividuell derartige Information in einem Programmspeicher hinterlegt sein, so dass für einen Patienten eine entsprechende Therapie durchgeführt werden kann. Typischerweise werden neben der Leistungsabgabe und dem Einschaltzustand auch die Dauer der gesamten Behandlung pro Therapiesitzung hinterlegt sein. Typische Bestrahlungszeiten sind im Bereich einiger Minuten, und betragen bis zu 20 min.

Gemäß einer weiteren Ausführungsform der Erfindung sind für die optischen Module der ersten LEDs und/oder der zweiten LEDs jeweils Blenden vorgesehen, die vorzugsweise als Lochscheibe ausgebildet sind. Ein Aufbau ohne Blenden ist allerdings ebenfalls möglich.

Die Lochscheibe kann dabei besonders bevorzugt an dem Bauteileträger montiert sein, und in anderen Ausführungsform auch das jeder LED zugeordnete optische Modul aufnehmen. Demnach wird ein kompakter Aufbau geschaffen, bei dem eine einfache und kostengünstige Montage eines großflächigen Therapiegeräts möglich ist.

Gemäß einer weiteren Ausführungsform der Erfindung weist das Therapiegerät ein vorzugsweise flächenhaftes Gehäuse auf, das an einer verstellbaren Halterung angebracht ist.

Das Therapiegerät benötigt aufgrund ihres kompakten Aufbaus nur relativ wenig Platz in einer Richtung, die der Abgabe des Lichts aus den LEDs entspricht. Neben den erwähnten Bauteilen ist zusätzlich nur noch ein Kühlkörper auf der Rückseite des Bauteileträgers notwendig, der die Wärme von den LEDs abführt, wobei in einer Ebene senkrecht dazu entsprechende Kühlvorrichtung beispielsweise in Form von Lüftern vorgesehen sein können. Insgesamt weist das Therapiegerät daher einen Aufbau auf, dessen Abmessungen in den Richtungen quer zur Abstrahlrichtung der LEDs deutlich größer als senkrecht dazu ist. Ein Therapiegerät in Form eines flächenhaften Gehäuses lässt sich relativ einfach über das zu behandelnde Areal bringen, wobei ein verstellbarer Arm während der Behandlung das Therapiegerät an der gewünschten Position hält.

Gemäß einer weiteren Ausführungsform der Erfindung weist das Gehäuse zum Abtransport der Wärme der ersten und/oder zweiten LEDs einen Lüfter auf, der typischerweise mit einem Filter, vorzugsweise mit einem Schwebstofffilter versehen ist.

Mittels des Schwebstofffilters kann dabei verhindert werden, Keime oder dergleichen innerhalb einer Behandlungsstätte wie z.B. einem Krankenhaus zu verschleppen.

Nachfolgend werden einige Ausführungsbeispiele anhand der Zeichnung näher erläutert. Es zeigen:
- Fig. 1: ein Therapiegerät gemäß einer ersten Ausführungsform der Erfindung in einer perspektivischen Seitenansicht,
- Fig. 2: eine Schnittansicht durch ein Therapiegerät,
- Fig. 3: in einer schematischen Darstellung die Abstrahlcharakteristik von LEDs eines erfindungsgemäßen Therapiegeräts,
- Fig. 4A/B: eine Zuordnung von ersten und zweiten Belichtungsfeldern eines erfindungsgemäßen Therapiegeräts zu einem gesamten Behandlungsgebiet in einer Draufsicht,
- Fig. 5: eine weitere Zuordnung von ersten und zweiten Belichtungsfeldern eines erfindungsgemäßen Therapiegeräts zu einem gesamten Behandlungsgebiet in einer Draufsicht,
- Fig. 6: ein Detail einer Anordnung von LEDs auf einem Bauteileträger eines erfindungsgemäßen Therapiegeräts in einer Draufsicht,
- Fig. 7: ein Detail einer Anordnung von LEDs auf einem Bauteleträger eines erfindungsgemäßen Therapiegeräts in einer Seitenansicht,
- Fig. 8 bis 10: ein erfindungsgemäßes Therapiegerät bei der Behandlung einer Wundstelle.

In den Figuren sind gleiche oder funktional gleich wirkende Bauteile mit den gleichen Bezugszeichen versehen.

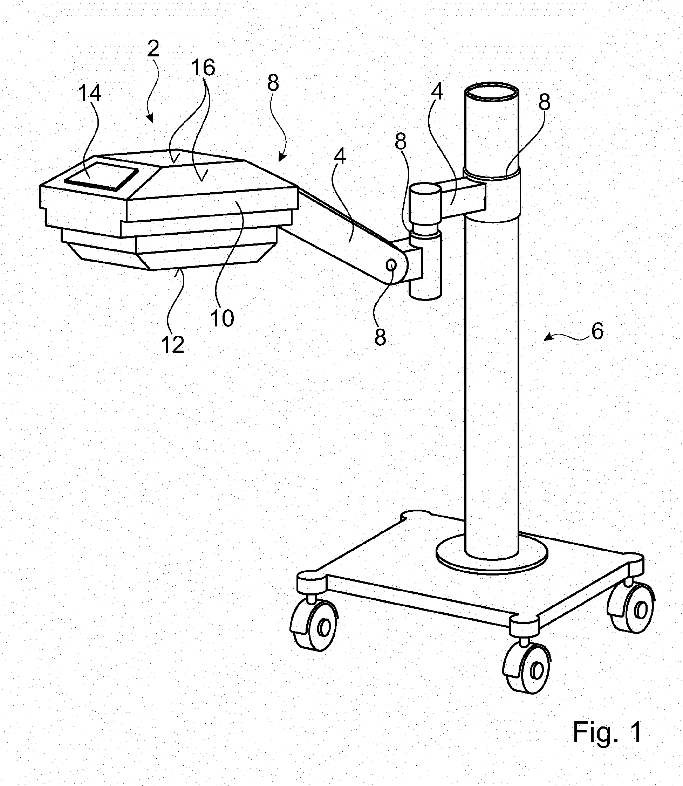

In Fig. 1 ist eine erste Ausführungsform eines erfindungsgemäßen Therapiegeräts 2 in einer perspektivischen Seitenansicht gezeigt. Das Therapiegerät 2 ist über mehrere verstellbare Halterungen 4 mit einem beweglichen Rollwagen 6 verbunden. Durch eine Vielzahl von Verstellmitteln 8 ist die Position des Therapiegeräts 2 veränderbar, so dass diese in Bezug auf einen Patienten entsprechend angeordnet werden kann. Das Therapiegerät 2 weist an einem Gehäuse 10 auf, das auf seiner Unterseite 12 eine Öffnung oder bevorzugt ein transparentes Fenster aufweist, so dass Licht unterschiedlicher Zentralen Wellenlängen aus dem Therapiegerät 2 zur Bestrahlung einer Oberfläche eines Gewebes herangezogen werden kann. Als Gewebe wird typischerweise Hautgewebe einer therapeutischen Behandlung unterzogen. Zur Kontrolle bzw. Steuerung der Behandlung ist eine berührungssensitive Anzeigeeinheit 14 vorgesehen, die typischerweise an einer Oberseite 16 des Therapiegeräts 2 angeordnet ist. Die Anzeigeeinheit 14 kann auch ausklappbar sein, so dass diese während der Behandlung von einem Bediener in eine gewünschte Position zur einfacheren Benutzung geschwenkt werden kann. Die Stromzuführung des Therapiegeräts 2 kann beispielsweise über die schwenkbaren Halterungen 4 und den Rollwagen 6 mittels entsprechend ausgebildeter Kabelkanäle durchgeführt werden.

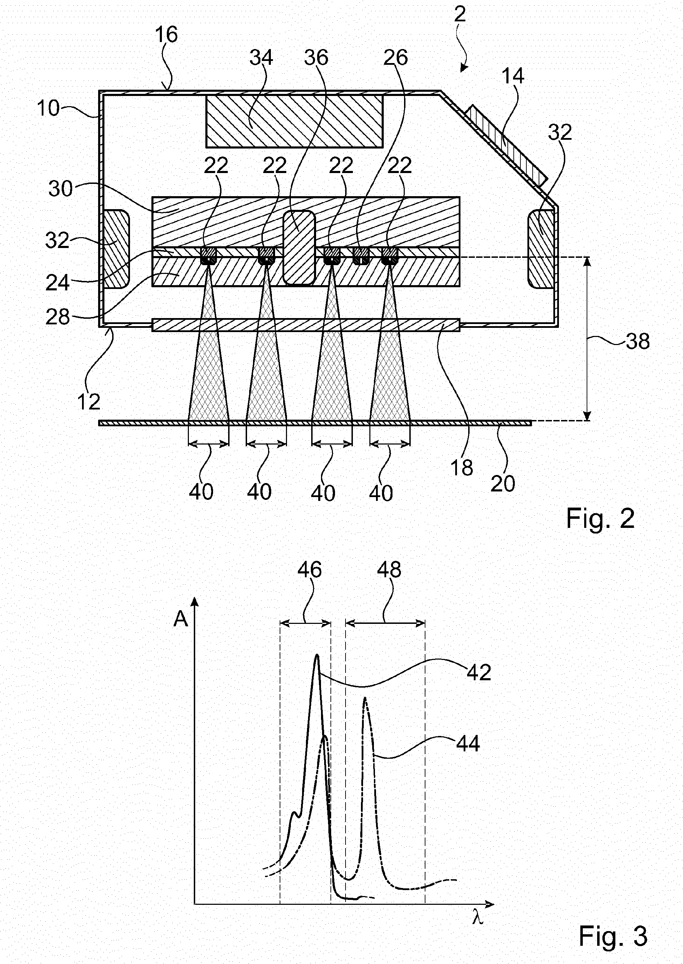

In Fig. 2 ist schematisch ein Schnitt durch das Therapiegerät 2 gezeigt. Man erkennt, dass das Therapiegerät 2 auf ihrer Unterseite 12 ein transparentes Fenster 18 aufweist, so dass Licht zur Bestrahlung einer Oberfläche 20 aus dem Therapiegerät 2 treten kann. Hierbei sind im Inneren des Therapiegeräts 2 mehrere erste LEDs 22 und wenigstens eine zweite LED 26 auf einem Bauteileträger 24 angeordnet. In Richtung der zu behandelnden Oberfläche 20 werden jeder ersten LED 22 und zweiten LED 26 jeweils ein optisches Modul 28 zugeordnet, das in Fig. 2 schematisch als eine optomechanische Einheit dargestellt ist. In Richtung der Oberseite 16 des Gehäuses 10 ist dem Bauteileträger 24 eine Wärmesenke 30 zugeordnet, die beim Betrieb der ersten LEDs 22 und der zweiten LEDs 26 entstehende Wärme abführen kann. Zum weiteren Abtransport der Wärme können auf gegenüberliegenden Seiten des Gehäuses 10 aktive Kühlvorrichtungen 32, beispielsweise in Form von Ventilatoren vorgesehen sein. Die Kühlvorrichtungen 32 können neben Ventilatoren auch Luftfilter enthalten um eine Verbreitung von Staub, Aerosole, Bakterien, Virenträgersubstanzen, usw., zu verhindern.

Desweiteren ist im Inneren des Gehäuses 10 ein Steuereinheit 34 vorgesehen, die die Ansteuerung sowohl der ersten LEDs 22 und der zweiten LEDs 26 als auch die Dateneingabe bzw. Datenausgabe über die Anzeigeeinheit 14 vornehmen kann. Auf der Anzeigeeinheit 14 können auf vorteilhafte Weise während des Betriebs des Therapiegeräts 2 auch Bilder einer ebenfalls auf dem Bauteileträger 24 angeordneten Kamera 36 dargestellt werden, so dass die relative Lage des Therapiegeräts 2 zur Oberfläche 20 des zu bestrahlenden Gewebes durch einen Behandler kontrolliert werden kann. Mittels eines nicht dargestellten Abstandssensors kann ein Abstand 38 bestimmt werden, der beispielsweise dem Behandler über die Anzeigeeinheit 14 mitgeteilt wird, wobei hier eventuell nur das Einhalten des korrekten vorgesehenen Abstands angezeigt werden muss.

Wie in Bezug auf Fig. 2 erläutert, sind erste LEDs 22 und zweite LEDs 26 vorgesehen, die für therapeutische Anwendungen auf der Oberfläche 20 oberhalb einer therapierelevanten Schwelle Licht emittieren. Hierbei ist es vorgesehen, dass die ersten LEDs 22 eine erste zentrale Wellenlänge aufweisen und die zweiten LEDs 26 eine zweite zentrale Wellenlänge verwenden. Die erste zentrale Wellenlänge ist dabei zur Durchführung einer photodynamischen Therapie vorgesehen, während die zweite zentrale Wellenlänge zur Durchführung einer regenerativen Photobiomodulationstherapie auf der Oberfläche 20 des Gewebes geeignet ist. Um nun beispielsweise ein infiziertes Wundareal entsprechend antimikrobiell mittels photodynamischer Therapie zu behandeln und außen liegende Bereiche zur Abschwellung von entzündetem Gewebe mittels regenerativer Photobiomodulationstherapie zu behandeln, liegen sowohl die ersten LEDs 22 als auch die zweiten LEDs 26 in einer Mehrfachanordnung vor, so dass innerhalb eines gemeinsamen Bestrahlungsgebiets eine Aufteilung in Belichtungsfeldern erfolgt, innerhalb derer ausgewählt werden kann, ob und welche der ersten LEDs 22 und der zweiten LEDs 26 aktiviert sein sollen. Somit kann eine zweidimensionale Bestrahlung in mehreren Belichtungsfeldern erfolgen, wobei für jedes Belichtungsfeld ein oder mehrere Therapieformen auswählbar sind.

Unter Bezugnahme auf Fig. 2 ist zu erkennen, dass die ersten LEDs 22 auf der Oberfläche 20 des zu behandelnden Gewebes jeweils Licht in erste Belichtungsfelder 40 abgeben. Für die zweiten LEDs 26 wurden entsprechend zweite Belichtungsfelder vorgesehen, die zur Vereinfachung jedoch nicht in Fig. 2 gezeigt sind. Es sei an dieser Stelle erwähnt, dass die typischerweise matrixförmige Anordnung der ersten LEDs 22 und der zweiten LEDs 26 nicht notwendigerweise mit gleicher Anzahl Zeilen und Spalten ausgebildet sein muss, so dass sich die oben beschriebenen Mehrfachanordnungen bezüglich der Größe der ersten Belichtungsfelder 40 und der zweiten Belichtungsfelder unterscheiden. Wichtig ist jedoch, dass sich die ersten Belichtungsfelder 40 und die zweiten Belichtungsfelder auf der Oberfläche 20 des Gewebes zu einem gemeinsamen Bestrahlungsgebiet überdecken.

Desweiteren ist Fig. 2 zu entnehmen, dass die Position der ersten LEDs 22 jeweils mittig in den ersten Belichtungsfeldern 40 gewählt ist. Dies trifft jedoch nur für den Fall zu, dass jedem ersten Belichtungsfeld 40 nur eine erste LED 22 zugeordnet ist. In manchen Anwendungsfällen können auch mehrere erste LEDs 22 in ein erstes Belichtungsfeld 40 Licht der ersten zentralen Wellenlänge abgeben. Aufgrund dieser Vorgehensweise ist es möglich, die Leistungsabgabe für jedes erste Belichtungsfeld zu erhöhen und ggf. die Homogenität der Leistungsverteilung zu verbessern. Eine ähnliche Vorgehensweise wäre auch in Bezug zu den zweiten LEDs 26 möglich. Ein weiterer für die Erfindung wesentlicher Punkt besteht darin, dass die optische Einheit 28 eine Abbildung der Emitterfläche der ersten LED 22 auf das jeweilige Belichtungsfeld 40 bewirkt, so dass die Ausdehnung des Bauteileträgers 24 dem zu behandelnden Gebiet auf der Oberfläche 20 entspricht. Insbesondere wird keine Aufweitung in Form einer Projektion über das zugehörige Belichtungsfeld hinaus vorgenommen. Ähnlich wird die Emitterfläche der zweiten LEDs 26 in das Belichtungsfeld 40 abgebildet.

Die Auswahl der zentralen Wellenlängen für die ersten LEDs 22 und den zweiten LEDs 26 folgt dabei dem gewünschten therapeutischen Effekt. Das Therapiegerät 2 eignet sich zur photodynamischen Therapie mit einem äußerlich applizierten Photosensibilisator und/beziehungsweise zur regenerativen Photobiomodulationstherapie, die unabhängig von einem äußerlich applizierten Photosensibilisator durchgeführt werden kann.

Bei der photodynamischen Therapie wird üblicherweise auf das zu behandelnde Areal ein Photosensibilisator (z.B. Methylenblau) aufgetragen, dessen Absorptionsspektrum in Abhängigkeit der Wellenlänge in Fig. 3 als Kurve 42 gezeigt ist. Zur regenerativen Photobiomodulationstherapie ist anhand der in den Zellen vorliegenden Bestandteile ebenfalls ein Absorptionsspektrum bekannt, das in Fig. 3 als Kurve 44 dargestellt ist. Man erkennt, dass für Licht der ersten zentralen Wellenlänge zur Durchführung einer antimikrobiellen photodynamischen Therapie ein wesentlicher Wellenlängenanteil im Wellenlängenbereich 46 gewählt werden sollte. Zur Durchführung einer regenerativen Photobiomodulationstherapie sollte Licht im Wellenlängenbereich 48 als zweite zentrale Wellenlänge bereitgestellt werden. Licht dieser zweiten zentralen Wellenlänge wird nicht vom Photosensibilisator absorbiert, weil die Kurve 42 im Wellenlängenbereich 48 kein Maximum aufweist, sodass die Photobiomodulationstherapie auch auf Arealen wo Photosensibilisator appliziert wurde stattfinden kann. Da die Kurve 44 sehr wohl auch im Wellenlängenbereich 48 ein deutliches Maximum aufweist, kann daher eine Trennung der verschiedenen Therapieformen über die Wellenlänge erfolgen.

Zur photodynamischen Therapie wird die erste zentrale Wellenlänge in einer Wellenlänge eingesetzt werden, welche auf das Absorptionsmaximum des verwendeten Photosensibilisators abgestimmt ist, typischerweise aber nicht ausschließend im visuellen Bereich. Die zweite zentrale Wellenlänge ist für eine effektive Photobiomodulationstherapie auf der durch Überlagerung mit den Absorptionsspektren der Metallzentren der mitochondrialen Cytochrom-C-Oxidase innerhalb der Zielzellen-Mitochondrien geeignet und befindet sich sowohl im roten als auch im nahen Infrarotbereich, typischerweise mit einer resp. Wellenlänge zwischen 650 nm und 700 nm und zwischen 780 nm und 850 nm. Anhand der in Fig. 3 gezeigten Strategie der Trennung von Therapiearten anhand der Absorptionsspektren lässt sich somit die geeigneten ersten LEDs 22 und zweiten LEDs 26 auswählen.

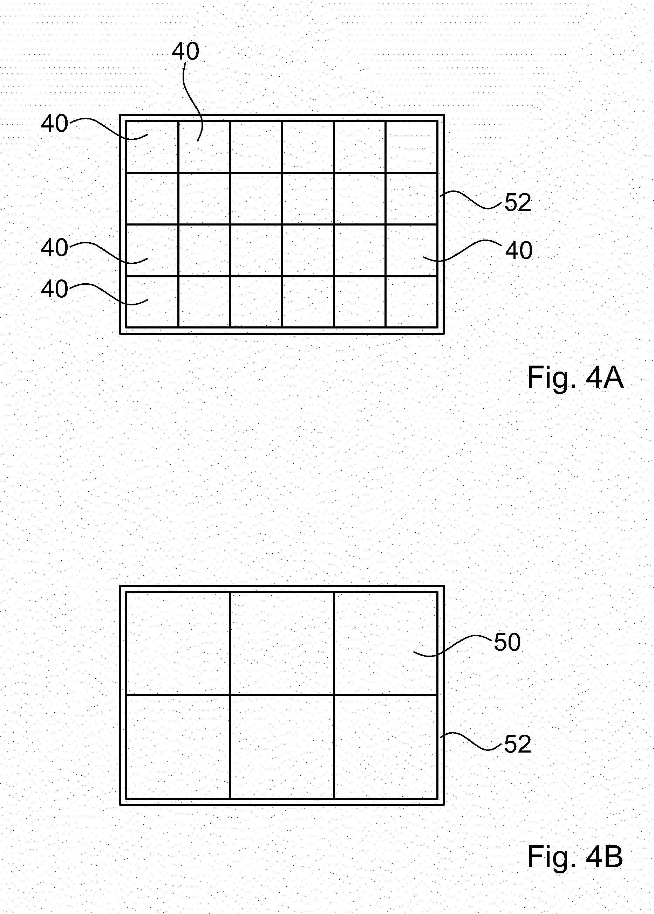

In Fig. 4A ist eine Zuordnung von ersten Belichtungsfeldern 40 und in Fig. 4B von zweiten Belichtungsfeldern 50 des Therapiegeräts 2 aus Fig. 1 zu dem gemeinsamen Bestrahlungsgebiet 52 in einer Draufsicht schematisch gezeigt. Zur besseren Veranschaulichung wurden die ersten Belichtungsfeldern 40 und zweiten Belichtungsfeldern 50 in getrennten Figuren dargestellt. Das gemeinsame Bestrahlungsgebiet 52 entspricht daher der Überlagerung aus Fig. 4A und 4B und wird in diesen Figuren als Außenrahmen dargestellt.

Man erkennt aus Fig. 4A, dass für die ersten Belichtungsfelder 40 eine Anordnung aus 4 × 6 Belichtungsfelder gewählt wurde. Für die zweiten Belichtungsfelder 50 wurde gemäß Fig. 4B eine Anordnung aus 2 × 3 Belichtungsfelder gewählt. Sowohl die ersten Belichtungsfelder 40 als auch die zweiten Belichtungsfelder 50 ergänzen sich zu einem homogenen Gebiet, das als gemeinsames Bestrahlungsgebiet 52 auf der Oberfläche 20 aus Fig. 2 des zu bestrahlenden Gewebes vorliegt. Für jedes des in Fig. 4A und 4B gezeigte erste Belichtungsfeld 40 bzw. zweite Belichtungsfeld 50 lässt sich nun anhand eines Therapieprogrammes festlegen, welche der ersten Belichtungsfelder 40 bzw. zweiten Belichtungsfelder 50 aktiviert sein sollen.

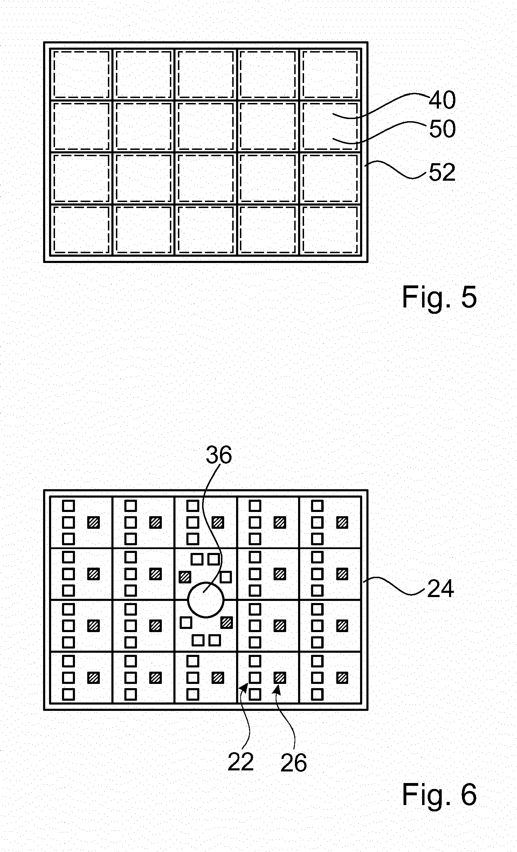

In Fig. 5 ist eine weitere Zuordnung der ersten Belichtungsfelder 40 bzw. zweiten Belichtungsfelder 50 zu einem gemeinsamen Bestrahlungsgebiet 52 gezeigt. Hier wird sowohl für die ersten Belichtungsfelder 40 als auch für die zweiten Belichtungsfelder 50 eine Zuordnung gewählt, die bezüglich der Zeilen und Spalten jeweils gleich ist. Im gezeigten Beispiel wird daher das gemeinsame Bestrahlungsgebiet 52 von 4 × 5 ersten Belichtungsfeldern 40 bzw. zweiten Belichtungsfeldern 50 homogen ausgeleuchtet. Wie auch im oberen Beispiel kann hier über das Therapieprogramm entschieden werden, welche der Belichtungsfelder 40 bzw. 50 aktiviert sein sollen. Das in Fig. 5 gezeigte Beispiel entspricht dabei einer Ausführungsform bzgl. der ersten Belichtungsfelder 40 wie in Fig. 2, wobei in Fig. 2 eine Schrägansicht in Richtung der vier nebeneinander liegenden Belichtungsfeldern 40 gewählt wurde.

In Fig. 6 ist in einer Art Bestückungsplan die Anordnung von ersten LEDs 22 und zweiten LEDs 26 auf dem Bauteileträger 24 gezeigt, um den Aufbau des Bauteileträgers 24 mit ersten LEDs 22 bzw. zweiten LEDs 26 auch bei Anwesenheit der optional vorgesehenen Kamera 36 nochmals zu verdeutlichen. Diese Anordnung wird überlagert durch Begrenzungslinien der ersten Belichtungsfelder 40 bzw. zweiten Belichtungsfelder 50 gemäß der Ausführungsform nach Fig. 5. Man erkennt, dass jedes erste bzw. zweite Belichtungsfeld 40, 50 mit jeweils drei ersten LEDs 22 bestückt ist. Zusätzlich ist für jedes erste bzw. zweite Belichtungsfeld 40/50 eine einzige zweite LED 26 vorgesehen. Die ersten LEDs 22 sind dabei streifenförmig angeordnet, wobei die beiden zentralen Belichtungsfelder eine andere Bestückung aufweisen, so dass an dieser Stelle Platz für die Kamera 36 geschaffen wird. Zusätzlich zu den ersten LEDs 22 bzw. zweiten LEDs 26 können auch weitere Weißlicht-LEDs (nicht in Fig. 6 gezeigt) verwendet werden, um ein kontrastreicheres Bild bei der optischen Inspektion mittels der Kamera 36 zu schaffen. Die Weißlicht-LEDs können am Gehäuse 10 an der Unterseite 12 montiert sein. In wiederum anderen Ausführungsformen ist es ebenso möglich, eine andere Anzahl erster LEDs 22 bzw. weitere zweite LEDs 26 vorzusehen.

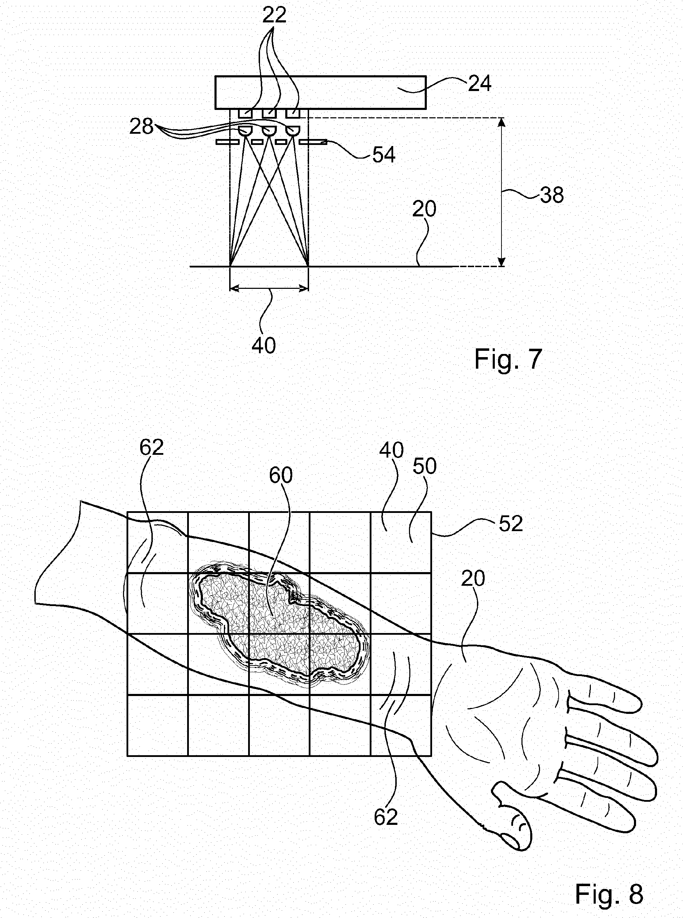

Das in Fig. 6 gezeigte Detail der Anordnung des Therapiegeräts 2 wird nachfolgend unter Bezugnahme auf Fig. 7 in einer Seitenansicht nochmals verdeutlicht. Man erkennt, dass die als asphärische Linsen ausgebildeten optischen Module 28 das Licht jeder ersten LED 22 auf den gesamten Bereich des ersten Belichtungsfelds 40 abbilden. Die optischen Module 28 können dabei als Kunststofflinsen ausgebildet sein, die in einem Spritzgussverfahren als ein gemeinsames Bauteil gefertigt werden können. Ein Mehrfach-Standardlinsen-Aufbau in einer optomechanischen Halterung ist ebenfalls möglich. An der Position des Lichtdurchtritts kann eine Lochblende 54 vorgesehen sein, die die Lichtausbreitung entsprechend beeinflusst. Aufgrund der geringeren Energie bei Durchführung einer regenerativen Photobiomodulationstherapie wird typischerweise pro Belichtungsfeld nur eine zweite LED 26 verwendet werden. Das Belichtungsfeld 40 überdeckt dabei in etwa eine Fläche von 1 cm² bis 45 cm², typischerweise von in etwa 9 cm², wobei zwischen Bauteileträger 24 im Therapiegerät und der Oberfläche 20 des zu bestrahlenden Gewebes ein Abstand 38 im Bereich von 30 mm bis 300 mm, typischerweise 150mm vorliegt.

Die Anwendung einer Therapiegerät 2 ist beispielhaft in Fig. 8 gezeigt. Man erkennt, dass das gemeinsame Bestrahlungsgebiet 52 die Oberfläche 20 überdeckt, wobei hier als zu bestrahlendes Gewebe ein Unterarm schematisch eingezeichnet ist. Auf der Oberfläche 20 ist mit dem Bezugszeichen 60 eine Wunde gekennzeichnet, die in ihren Außenbereichen von Gebieten mit entzündlichen Hautbereichen 62 umgeben ist.

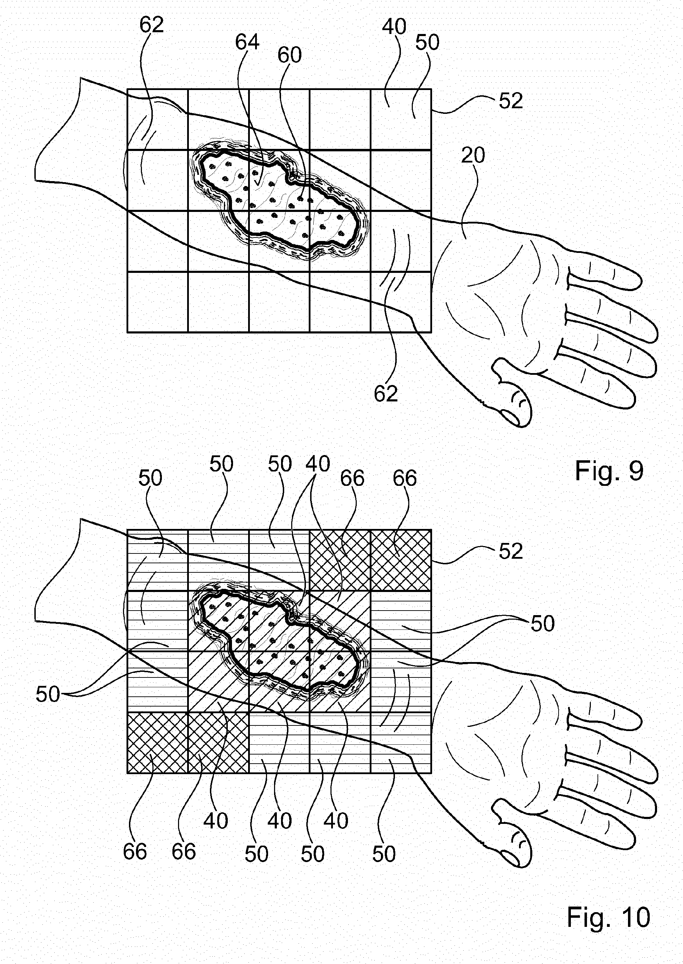

Vor Verwendung der Therapiegerät 2 wird zunächst, wie in Fig. 9 gezeigt ist, die Wunde 60 mit einem Photosensibilisator 64 überdeckt. Die Lampe wird mittels der in Fig. 1 beschriebenen verstellbaren Halterungen 4 über das Gebiet der Wunde 60 gebracht. Zur Kontrolle kann dazu die Kamera 36 bzw. deren Darstellung der aufgenommenen Bilder, ggf. ergänzt mit den Messdaten eines Abstandsensors zur Aufzeichnung des Abstands 38, auf der Anzeigeeinheit 14 verwendet werden. Zusätzlich können dazu die Weißlicht-LEDs aktiviert sein, wie oben beschrieben.

Fig. 10 zeigt nun die Auswahl der entsprechenden Therapieformen. Bereiche, die kein Gewebe innerhalb des gemeinsamen Bestrahlungsgebiets 52 überdecken, werden ausgeklammert, so dass an dieser Stelle überhaupt keine Lichtenergie abgegeben wird. Diese sind innerhalb des gemeinsamen Bestrahlungsgebiets 52 mit dem Bezugszeichen 66 gekennzeichnet. Erste Belichtungsfelder 40, die die mit Photosensibilisator 64 gefärbten Wundfläche 60 überdecken, werden so aktiviert, dass dort eine antimikrobielle photodynamische Therapie durchgeführt werden kann. Die Belichtungsfelder, welche lediglich die Hautreizungsbereiche 62 überdecken, werden vom Licht der zweiten LEDs 26 bestrahlt, so dass eine regenerative Photobiomodulationstherapie durchgeführt wird. Es sei an dieser Stelle erwähnt, dass es auch möglich ist, die zweiten LEDs 26 auch in denjenigen Bereichen zu aktivieren, in denen die ersten LEDs 22 eingeschaltet sind.

Mittels der gezeigten Ausführungsformen der erfindungsgemäßen Therapiegerät 2 lässt sich somit eine großflächige Behandlung der Wunde 60 durchführen, wobei hier wenigstens zwei unterschiedliche Therapieformen ortsaufgelöst ausgewählt werden können.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar.

### Liste der Bezugszeichen:

- 2: Therapiegerät
- 4: Halterungen
- 6: Rollwagen
- 8: Verstellmitteln
- 10: Gehäuse
- 12: Unterseite
- 14: Anzeigeeinheit
- 16: Oberseite
- 18: Fenster
- 20: Oberfläche
- 22, 26: erste, zweite LEDs
- 24: Bauteileträger
- 28: optisches Modul
- 30: Wärmesenke
- 32: Kühlvorrichtungen
- 34: Steuereinheit
- 36: Kamera
- 38: Abstand
- 40, 50: erstes, zweites Belichtungsfeld
- 42, 44: erste, zweite Kurve
- 46, 48: erster, zweiter Wellenlängenbereich
- 52: Bestrahlungsgebiet
- 54: Lochblende
- 60: Wunde
- 62: Hautbereich
- 64: Photosensibilisator

## Patentansprüche

1. Therapiegerät zur Bestrahlung einer Oberfläche (20) eines Gewebes, insbesondere Hautgewebe, das in einer Mehrfachanordnung mehrere erste LEDs (22) mit einer ersten zentralen Wellenlänge und mehrere zweite LEDs (26) mit einer zweiten zentralen Wellenlänge aufweist, wobei die erste zentrale Wellenlänge zur Durchführung einer ersten Phototherapieart und die zweite zentrale Wellenlänge zur gleichzeitiger Durchführung einer zweiten Phototherapieart auf dem Gewebe geeignet sind, wobei die mehreren ersten LEDs (22) und die mehreren zweiten LEDs (26) separat bezüglich ihrer Leistung und Bestrahlungsdauer als elektrische Betriebsgrößen steuerbar sind ;
wobei die ersten LEDs (22) und die zweiten LEDs (26) auf einem Bauteileträger (24) angeordnet sind, dessen Außenabmessung im Wesentlichen der zu bestrahlenden Oberfläche (20) auf dem Gewebe entspricht und bei dem die Leistungsabgabe, die Zeitdauer und der Einschaltzustand der ersten LEDs (22) und/oder der zweiten LEDs (26) in einem Programmspeicher als Therapiedaten hinterlegt sind, so dass die die ersten LEDs (22) und/oder die zweiten LEDs (26) bezüglich ihrer Leistungsabgabe nach einem vorgegebenen Programm steuerbar sind;
**gekennzeichnet dadurch, dass**:
jeder ersten LED und jeder zweiten LED jeweils ein optisches Modul zur lateralen Begrenzung der Abstrahlung in einem Belichtungsfeld zugeordnet ist, so dass den mehreren ersten LEDs erste Belichtungsfelder und den mehreren zweiten LEDs zweite Belichtungsfelder zugeordnet sind,
wobei sich die ersten Belichtungsfelder und die zweiten Belichtungsfelder auf der Oberfläche des Gewebes zu einem gemeinsamen Bestrahlungsgebiet ergänzen, derart, dass sich sowohl die ersten Belichtungsfelder zum gemeinsamen Bestrahlungsgebiet ergänzen als auch sich die zweiten Belichtungsfelder zum gemeinsamen Bestrahlungsgebiet ergänzen, so dass jedes erste Belichtungsfeld mit einer Leistungs- und Energiedichte oberhalb einer Therapieschwelle indiziert für die erste Phototherapieart und jedes zweite Belichtungsfeld mit einer Leistungs- und Energiedichte oberhalb einer Therapieschwelle indiziert für die zweite Phototherapieart mit möglichst homogenen Leistungsverteilungen bestrahlbar ist,
wobei für jedes erste Belichtungsfeld und jedes zweite Belichtungsfeld individuell auswählbar ist, ob die erste LED oder ersten LEDs beziehungsweise die zweite LED oder zweiten LEDs, die dem Belichtungsfeld zugeordnet sind, aktiviert werden sollen oder nicht.

2. Therapiegerät nach Anspruch 1, bei dem die erste Phototherapieart eine regenerative Photobiomodulationstherapie oder eine photodynamische Therapie und bei dem die zweite Phototherapieart eine weitere regenerative Photobiomodulationstherapie ist.

3. Therapiegerät nach Anspruch 1 oder 2, bei dem für jedes erste Belichtungsfeld (40) mehrere erste LEDs (22) vorgesehen sind.

4. Therapiegerät nach Anspruch 1 bis 3, bei dem die ersten Belichtungsfelder (40) und die zweiten Belichtungsfelder (50) in Form einer Mehrfachanordnung ausgebildet sind.

5. Therapiegerät nach einem der Ansprüche 1 bis 4, bei dem an ausgewählten Belichtungsfeldern, insbesondere im Bereich der zentralen Belichtungsfeldern bezüglich der zu bestrahlenden Oberfläche (20), die ersten LEDs (22) und die zweiten LEDs (26) so angeordnet sind, dass ein Freiraum im Bauteilträger (24) gebildet ist, vorzugsweise zum Anbringen einer Kamera (36).

6. Therapiegerät nach Anspruch 5, bei dem eine an einer Oberseite (16) des Therapiegeräts (2) vorzugsweise ausklappbare Anzeigeeinheit (14) vorgesehen ist, die Daten der Kamera (36) darstellt.

7. Therapiegerät nach einem der Ansprüche 1 bis 6, bei dem das optische Modul (28) der ersten (22) LEDs und/oder zweiten LEDs (26) eine asphärische Linse aufweist.

8. Therapiegerät nach einem der Ansprüche 1 bis 7, bei dem das Gehäuse (10) zum Abtransport der Wärme der ersten LEDs (22) und/oder zweiten LEDs (26) einen Lüfter als Kühlvorrichtung (32) aufweist.

9. Therapiegerät nach Anspruch 8, bei dem der Lüfter mit einem Filter, vorzugsweise mit einem Schwebstofffilter versehen ist.

10. Therapiegerät nach einem der Ansprüche 1 bis 9, bei dem eine zuschaltbare Lichtquelle zur optischen Kontrolle der zu behandelnden Oberfläche (20) vorgesehen ist.

11. Therapiegerät nach Anspruch 10, bei dem die zuschaltbare Lichtquelle als weitere LED benachbart zu einer der ersten LEDs (22) und/oder zweiten LEDs (26) im Gehäuse angeordnet ist.

12. Therapiegerät nach Anspruch 10, bei dem die zuschaltbare Lichtquelle als weitere LED am Gehäuse (10) angeordnet ist.

13. Therapiegerät nach einem der Ansprüche 1 bis 12, das einen Abstandssensor zur Messung eines Abstands (38) zur Oberfläche (20) des zu behandelnden Gewebes aufweist.

14. Therapiegerät nach einem der Ansprüche 1 bis 13, bei dem für die optischen Module (28) der ersten LEDs (22) und/oder der zweiten LEDs (26) jeweils Blenden vorgesehen sind, die vorzugsweise als Lochscheibe ausgebildet sind.

15. Therapiegerät nach einem der Ansprüche 1 bis 14, das ein vorzugsweise flächenhaftes Gehäuse (10) aufweist, das an einer verstellbaren Halterung (4) angebracht ist.

## Claims

1. Therapy device for irradiating a surface (20) of a tissue, in particular skin tissue, which comprises, in a multiple arrangement, a plurality of first LEDs (22) having a first central wavelength and a plurality of second LEDs (26) having a second central wavelength, the first central wavelength being suitable for carrying out a first type of phototherapy on the tissue and the second central wavelength being suitable for simultaneously carrying out a second type of phototherapy on the tissue, it being possible to control the plurality of first LEDs (22) and the plurality of second LEDs (26) separately with respect to their power and irradiation duration as electrical operating variables;
the first LEDs (22) and the second LEDs (26) being arranged on a component carrier (24), the outer dimensions of which substantially correspond to the surface (20) of the tissue to be irradiated, and the power output, the duration and the switched-on state of the first LEDs (22) and/or the second LEDs (26) being stored in a program memory as therapy data such that the first LEDs (22) and/or the second LEDs (26) can be controlled with regard to their power output according to a predetermined program;
**characterized in that**:
an optical module for laterally limiting the radiation in an exposure field is assigned to each first LED and each second LED such that first exposure fields are assigned to the plurality of first LEDs and second exposure fields are assigned to the plurality of second LEDs,
the first exposure fields and the second exposure fields combining with one another on the surface of the tissue to form a common irradiation region such that both the first exposure fields combine with one another to form the common irradiation region and the second exposure fields combine with one another to form the common irradiation region such that each first exposure field having a power and energy density above a therapy threshold indicated for the first type of phototherapy and each second exposure field having a power and energy density above a therapy threshold indicated for the second type of phototherapy can be irradiated with the most homogeneous power distributions possible,
it being possible to individually select, for each first exposure field and each second exposure field, whether or not the first LED or first LEDs or the second LED or second LEDs assigned to the exposure field should be activated.

2. Therapy device according to claim 1, wherein the first type of phototherapy is a regenerative photobiomodulation therapy or a photodynamic therapy and wherein the second type of phototherapy is a further regenerative photobiomodulation therapy.

3. Therapy device according to either claim 1 or claim 2, wherein a plurality of first LEDs (22) are provided for each first exposure field (40).

4. Therapy device according to claims 1 to 3, wherein the first exposure fields (40) and the second exposure fields (50) are in the form of a multiple arrangement.

5. Therapy device according to any of claims 1 to 4, wherein the first LEDs (22) and the second LEDs (26) are arranged at selected exposure fields, in particular in the region of the central exposure fields with respect to the surface (20) to be irradiated, such that a space is formed in the component carrier (24), preferably for attaching a camera (36).

6. Therapy device according to claim 5, wherein a display unit (14) which can preferably be folded out and displays data from the camera (36) is provided on an upper face (16) of the therapy device (2).

7. Therapy device according to any of claims 1 to 6, wherein the optical module (28) of the first LEDs (22) and/or second LEDs (26) comprises an aspheric lens.

8. Therapy device according to any of claims 1 to 7, wherein the housing (10) comprises a fan as a cooling device (32) for removing the heat from the first LEDs (22) and/or second LEDs (26).

9. Therapy device according to claim 8, wherein the fan is provided with a filter, preferably with a high-efficiency particulate air filter.

10. Therapy device according to any of claims 1 to 9, wherein a switchable light source is provided for optically controlling the surface (20) to be treated.

11. Therapy device according to claim 10, wherein the switchable light source is arranged in the housing as a further LED so as to be adjacent to one of the first LEDs (22) and/or second LEDs (26).

12. Therapy device according to claim 10, wherein the switchable light source is arranged on the housing (10) as a further LED.

13. Therapy device according to any of claims 1 to 12, which has a distance sensor for measuring a distance (38) to the surface (20) of the tissue to be treated.

14. Therapy device according to any of claims 1 to 13, wherein apertures are provided for the optical modules (28) of the first LEDs (22) and/or the second LEDs (26), which apertures are preferably designed as perforated disks.

15. Therapy device according to any of claims 1 to 14, which has a preferably planar housing (10) which is attached to an adjustable holder (4).

## Revendications

1. Appareil de thérapie destiné à irradier une surface (20) d'un tissu, en particulier du tissu cutané, qui présente plusieurs premières DEL (22) comportant une première longueur d'onde centrale et plusieurs secondes DEL (26) comportant une seconde longueur d'onde centrale dans un agencement multiple, la première longueur d'onde centrale étant adaptée pour la mise en oeuvre d'un premier type de photothérapie et la seconde longueur d'onde centrale étant adaptée pour la mise en oeuvre simultanée d'un second type de photothérapie sur le tissu, les plusieurs premières DEL (22) et les plusieurs secondes DEL (26) pouvant être commandées séparément en ce qui concerne leur puissance et leur durée d'irradiation en tant que grandeurs de fonctionnement électriques ;
les premières DEL (22) et les secondes DEL (26) étant disposées sur un support de composants (24), dont les dimensions extérieures correspondent sensiblement à la surface (20) à irradier sur le tissu et la puissance de sortie, la durée et l'état d'allumage des premières DEL (22) et/ou des secondes DEL (26) étant enregistrés dans une mémoire de programme en tant que données de thérapie, de sorte que les premières DEL (22) et/ou les secondes DEL (26) peuvent être commandées, en ce qui concerne leur puissance de sortie, selon un programme prédéfini ;
**caractérisé en ce que** :
un module optique destiné à limiter latéralement le rayonnement dans un champ d'exposition est attribué respectivement à chaque première DEL et à chaque seconde DEL, de sorte que des premiers champs d'exposition sont attribués aux plusieurs premières DEL et des seconds champs d'exposition sont attribués aux plusieurs secondes DEL,
les premiers champs d'exposition et les seconds champs d'exposition se complétant sur la surface du tissu pour former une zone d'irradiation commune, de telle sorte que les premiers champs d'exposition se complètent pour former une zone d'irradiation commune et les seconds champs d'exposition se complètent également pour former une zone d'irradiation commune, de sorte que chaque premier champ d'exposition comportant une densité de puissance et d'énergie au-dessus d'un seuil de thérapie indiqué pour le premier type de photothérapie et chaque second champ d'exposition comportant une densité de puissance et d'énergie au-dessus d'un seuil de thérapie indiqué pour le second type de photothérapie peut être irradié avec des distributions de puissance les plus homogènes possibles,
chaque premier champ d'exposition et chaque second champ d'exposition pouvant être sélectionné individuellement si la première DEL ou les premières DEL ou la seconde DEL ou les secondes DEL, auxquelles sont attribués les champs d'exposition, doivent être activées ou non.

2. Appareil de thérapie selon la revendication 1, dans lequel le premier type de photothérapie est une thérapie de photobiomodulation régénérative ou une thérapie photodynamique et dans lequel le second type de photothérapie est une autre thérapie de photobiomodulation régénérative.

3. Appareil de thérapie selon la revendication 1 ou 2, dans lequel plusieurs premières DEL (22) sont prévues pour chaque premier champ d'exposition (40).

4. Appareil de thérapie selon les revendications 1 à 3, dans lequel les premiers champs d'exposition (40) et les seconds champs d'exposition (50) sont réalisés sous la forme d'un agencement multiple.

5. Appareil de thérapie selon l'une des revendications 1 à 4, dans lequel les premières DEL (22) et les secondes DEL (26) sont disposées au niveau de champs d'exposition sélectionnés, en particulier dans la zone des champs d'exposition centraux par rapport à la surface (20) à irradier, de sorte qu'un espace libre est formé dans le support de composants (24), de préférence pour la fixation d'une caméra (36).

6. Appareil de thérapie selon la revendication 5, dans lequel une unité d'affichage (14), de préférence dépliable, qui affiche les données de la caméra (36), est prévue sur une face supérieure (16) de l'appareil de thérapie (2).

7. Appareil de thérapie selon l'une des revendications 1 à 6, dans lequel le module optique (28) des premières DEL (22) et/ou des secondes DEL (26) présente une lentille asphérique.

8. Appareil de thérapie selon l'une des revendications 1 à 7, dans lequel le boîtier (10) présente un ventilateur en tant que dispositif de refroidissement (32) pour évacuer la chaleur des premières DEL (22) et/ou des secondes DEL (26).

9. Appareil de thérapie selon la revendication 8, dans lequel le ventilateur est pourvu d'un filtre, de préférence d'un filtre à particules en suspension.

10. Appareil de thérapie selon l'une des revendications 1 à 9, dans lequel une source lumineuse commutable est prévue pour la commande optique de la surface (20) à traiter.

11. Appareil de thérapie selon la revendication 10, dans lequel la source lumineuse commutable est disposée en tant qu'autre DEL adjacente à l'une des premières DEL (22) et/ou des secondes DEL (26) dans le boîtier.

12. Appareil de thérapie selon la revendication 10, dans lequel la source lumineuse commutable est disposée en tant qu'autre DEL sur le boîtier (10).

13. Appareil de thérapie selon l'une des revendications 1 à 12, qui présente un capteur de distance destiné à mesurer une distance (38) par rapport à la surface (20) du tissu à traiter.

14. Appareil de thérapie selon l'une des revendications 1 à 13, dans lequel des ouvertures sont respectivement prévues pour les modules optiques (28) des premières DEL (22) et/ou des secondes DEL (26) et sont de préférence conçues comme des disques à trous.

15. Appareil de thérapie selon l'une des revendications 1 à 14, qui présente un boîtier (10), de préférence plat, fixé sur un support réglable (4).
